# EUROPEAN PATENT SPECIFICATION

(11) **EP 3 545 947 B1**
(45) Date of publication and mention of the grant of the patent: **01.11.2023**
(21) Application number: 19163863.4
(22) Date of filing: 19.03.2019
(51) Int. Cl.: A61K 8/9789, A61K 8/60, A61K 36/28, A61K 36/63, A61P 13/00, A61P 9/00

(54) **COMPOSITION FOR THE REDUCTION OF PLASMA LEVELS OF URIC ACID**
ZUSAMMENSETZUNG ZUR REDUZIERUNG DES PLASMASPIEGELS VON HARNSÄURE
COMPOSITION POUR LA RÉDUCTION DES NIVEAUX DE PLASMA DE L'ACIDE URIQUE

(30) Priority: 26.03.2018 IT 201800003917
(43) Date of publication of application: 02.10.2019
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Longoni, Alessandra

(56) References cited:
- CN-A- 106 880 665
- CN-A- 107 308 339
- CN-A- 107 468 998
- US-A1- 2009 304 831
- LE CHAMP DE L'AIR: "Detox Herbal Tea", GNPD, MINTEL, 1 February 2015 (2015-02-01), XP002787084,
- Maria S Gião ET AL: "Protection of deoxyribose and DNA from degradation by using aqueous extracts of several wild plants", JOURNAL OF THE SCIENCE OF FOOD AND AGRICULTURE, vol. 88, no. 4, 1 January 2008 (2008-01-01), pages 633-640, XP055529631, GB ISSN: 0022-5142, DOI: 10.1002/jsfa.3128

## Description

The present invention relates to a composition comprising an association of extract of at least a plant belonging to the genus *Cynara* and extract of at least a plant of the genus *Fraxinus* for the prevention and/or the treatment of hyperuricemia and of diseases related to it. Said composition is particularly effective in reducing plasma levels of uric acid thanks to the synergistic action of its components.

### Background of the invention

Recent epidemiological surveys suggested that, in the last decades, the prevalence and incidence of hyperuricemia and gout have risen in many Countries, mainly due to changes in eating habits and the increase in comorbidity prevalence, such as hypertension, obesity, metabolic syndrome, type 2 diabetes and kidney failure, which promote hyperuricemia. Between other factors responsible for the increase of gout prevalence, there are drugs such as salicylate and diuretics for the treatment of cardiovascular diseases.

Currently, the gout is one of the main causes of inflammatory arthritis in industrialized Countries. It is a disease associated to disability, low life quality and increased mortality, therefore it represents a constantly growing problem of public health. Recent experimental evidences have shown that high plasma concentrations of uric acid, with or without gout, are linked to cardiovascular diseases such as hypertension, coronary heart diseases, peripheral vascular diseases and ischemia.

Despite our comprehension of the pathophysiological role of hyperuricemia in gout and other diseases, as well as the availability of different therapeutic options, the treatment of patients suffering from gout is not optimal. In fact, the management of gouty patients implies the control of risk factors related to hyperuricemia, the rapid and effective control of acute attacks and the prevention of tophi and kidney stones formation in chronic patients.

Gout is caused by an excess of uric acid in the blood that, due to the over-saturation, causes the deposition of monosodium urate crystals in joints and soft tissues. Monosodium urate crystals represent pro-inflammatory stimuli which can trigger, amplify and sustain the inflammatory response. Because of this, the therapeutic target for the treatment of acute gout is the suppression of the expression, secretion and signaling of pro-inflammatory cytokines.

In case of acute attacks of gout associated with moderate pain (< 6 on the VAS scale from 1 to 10), monotherapy with steroidal and not steroidal anti-inflammatory drugs or with colchin is recommended. To maximize its effectiveness, the therapy with colchin should begin within 24 hours from the acute attack. As regards the anti-inflammatory drugs, a study has shown that prednisolone and naproxen have the same effectiveness in the treatment of gout. In more severe forms of gout, characterized by intense pain and often by a polyarticular manifestation of the disease, it is necessary to resort to polytherapy (colchin with FANS or glucocorticoids) or intra-articular administration of steroidal anti-inflammatory drugs.

For many decades, uric acid has been considered exclusively as a powerful antioxidant agent able to act as intracellular "scavenger" for the excess of oxygen free radicals present in conditions of increased oxidative stress. Actually, several clinical and experimental data suggest that when plasma levels of uric acid reach supraphysiological values, it is able to explicate pro-oxidant actions, inducing an increased production of oxygen free radicals and a series of other alterations which are potentially harmful for the vascular wall and other tissues. In fact, several epidemiological data have suggested that chronical hyperuricemia was able to predict the development of arterial hypertension independently from classical risk factors.

In a large observational study, including more than 8,000 subjects, it has been observed that moderately high levels of uricaemia were independently associated to a higher frequency of atherosclerotic plaques at carotid level especially in subjects without metabolic syndrome. The same authors have also shown that hyperuricemia was associated to a greater aortic vascular stiffness, independently from concurrent cardiovascular risk factors.

The main goal for the management of gout is to lower serum urate levels, in order to favor the dissolution of existing crystals and prevent the formation of new crystals. This goal is reached by maintaining the plasmatic concentration of uric acid below the saturation point of monosodium urate (<6 mg/dL or <360 µmol/L).

The main pharmacological therapies for gout provide for the use of the following drugs:
1. For the acute phase:
   a. Colchin;
   b. Non steroidal anti-inflammatory drugs.
2. For the prevention:
   a. Allopurinol;
   b. Febuxostat;
   c. Probenecid.

These drugs are not always effective for the prevention or for the treatment of hyperuricemia and gout and are associated to numerous side effects such as nausea, vomit, diarrhea, alopecia, myotonia, leukopenia, thrombocytosis, agranulocytosis, aplastic anemia, thrombocytopenia, foruncolosis, liver function alterations, headache, rashes and edema.

"Detox Herbal Tea" by "Le Champ de L'Air" (Mintel GNPD Record ID 2997362) for preparation with hot water contains artichoke leaf and ash tree leaf. US2009/304831 discloses a laxative composition which comprises artichoke extract and European ash extract. CN106880665 discloses Cynara scolymus leaf as part of a Chinese medical detoxification composition useful for treating uremia.

Therefore, there is still the need of alternative pharmaceutical compositions effective in the reduction of serum levels of uric acid and in the treatment of diseases associated to it, which do not present the side effects and disadvantages of currently available therapies.

### Detailed Description of the Invention

The inventors of the present invention have now surprisingly found that the association of some plant extracts such as extract of at least a plant belonging to the genus *Cynara* and extract of at least a plant belonging to the genus *Fraxinus* represents a valid alternative to clinically available synthetic active ingredients.

In particular, the association of the present invention comprises extract of *Cynara cardunculus* subsp. *scolymus* and/or *Cynara cardunculus var. altilis* and extract of *Fraxinus excelsior* as defined in claim 1.

Moreover, the association of the present invention results in an unexpected synergistic effect of its components.

### Cynara cardunculus

The Artichoke (*Cynara cardunculus* var. *scolymus*) is a variety of a thistle species mainly cultivated for food purposes. It is a plant native to Mediterranean, Northern Africa and Southern Europe, and it is cultivated in all the subtropical regions.

It is a perennial herbaceous plant, full of thorns, in average 1.5 m high. The leaves are large, alternated and deeply toothed. The high violet flowers are grouped in large heads with a diameter of 10-15 cm and supported by grooved and branched stems, with sessile leaves.

The drug consists of dried leaves. The latter have a length up to 50 cm and are large up to 25 cm, with 1 cm of thickness. The leaves form flat and lanceolate segments, with roughly toothed margins. The upper surface presents a brown-greenish color, the lower surface presents a grey color and it is extensively covered with trichomes. The smell of the drug is tenuous, slightly acid, the taste initially salty, then bitter. The artichoke is widely used in traditional medicine for the treatment of anemia, diabetes, pyrexia, gout, rheumatism and kidney stones. Clinical data have supported the use of artichoke extract in the treatment of digestive disorder (such as dyspepsia, meteorism, flatulence, nausea, vomit and stomachache) and hypercholesterolemia. Other uses, described in pharmacopoeias of all the world, are related to the treatment of atherosclerosis, kidney dysfunction and irritable bowel syndrome.

The drug contains up to 6% of phenolic acids, including 1-O-caffeoylquinic acid, 3-O-caffeoylquinic acid (also known as chlorogenic acid), caffeic acid, 4-O-caffeoylquinic acid and 1,5-di-O-caffeoylquinic acid (also known as cynarin). Other chemical compounds present up to 5% in the drug are sesquiterpene lactones, such as cynaropicrin, dihydrocynaropicrin, grosheimin and derivatives thereof. Less represented compounds are flavonoids (0.35-0.75%), including scolymoside, cynaroside and cynarotrioside. Flavonoids are a group of substances with different biological and pharmacological properties such as antibacterial, antiviral, antioxidant and anti-mutagenic activity, as well as the ability to inhibit different enzymatic systems. In fact, it has been reported that flavonoids are able to inhibit xanthine oxidase and the action of the superoxide radical contributing to solve not only the problems related to gout, but also the ones linked to oxidative stress.

In fact, the xanthine oxidase enzyme catalyzes the oxidation reactions from hypoxanthine to xanthine and from xanthine to uric acid, according to the following reactions:

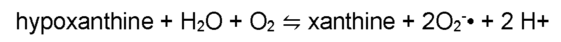

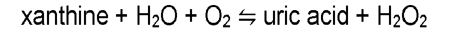

Some of the flavonoids present in the extract of artichoke, such as luteolin and chlorogenic acid, have proven themselves to be particularly effective in the inhibition of xanthine oxidase.

### Fraxinus excelsior

Ash Tree (*Fraxinus excelsior L*.) is a plant belonging to the family of Oleaceae, which naturally grows in the temperate regions of Europe and Asia, and in Morocco.

Ash Tree can grow up to 20-35 m high. It is found in the whole Italian peninsula, less sporadically in the central-northern Apennines, where it flourishes in the phytoclimatic areas of Castanetum, Fagetum and more rarely of Lauretum. It has a straight and cylindrical trunk initially with a smooth and olive-colored, subsequently gray-brownish and longitudinally cracked bark; the buds are velvety and blackish in color; it has large composite deciduous leaves imparipennate formed by 4-7 pairs of opposite sessile and minutely serrated leaflets dark and bright green in color on the upper side lighter on the lower one; the flowers, hermaphrodites, are collected in axillary panicles inflorescences and they are small, greenish in color and they appear before the leaves; they are calyx and corolla free with very short stamens surmounted by a globular anther dark purple in color; the fruits are long-shaped samaras of variable shape with a rounded or truncated base, with a single seed, gathered in hanging clusters.

It is a plant associated to a considerable traditional use, as food, condiment and herbal remedy to improve different pathological conditions. There are evidences in the use of Ash Tree seeds in Europe since the Middle Ages. The aqueous extract of Ash Tree seeds is recognized as effective in the treatment of hyperglycemia and diabetes from the traditional medicine of different Countries. This plant is known in the traditional medicine also for other beneficial effects on the organism: antioxidant, anti-inflammatory, anti-rheumatic, analgesic and antipyretic.

In the literature numerous studies on the hypoglycemic, hypolipidemic and diuretic effect of the Ash Tree exist. However, the experimental evidences are conflicting. In a study on the diuretic activity of the aqueous extract of Ash Tree, conducted on rats, it has emerged that the extract does not increase the urinary volume in a statistically significant manner, but it increases the sodium excretion with urine.

The drug contains active ingredients such as ursolic acid, benzoquinone, mannitol, tannins, phenolic acids, coumarins (fraxoside and esculoside), flavonoids. (rutoside), and ascorbic acid.

Therefore, an object of the present invention is a composition comprising the association of extract of at least a plant belonging to the genus *Cynara* and extract of at least a plant belonging to the genus *Fraxinus* in admixture with one or more suitable acceptable carriers.

In particular, the composition object of the present invention comprises the association extract of *Cynara cardunculus* subsp. *scolymus* and/or *Cynara cardunculus var. altilis* and extract of *Fraxinus excelsior,* possibly in admixture with one or more suitable acceptable carriers.

The extract of the plant belonging to the genus *Cynara* can be present in an amount comprised between 1 mg and 5000 mg, preferably between 50 mg and 4000 mg, still more preferably between 100 mg and 2000 mg.

The extract of the plant belonging to the genus *Fraxinus* can be present in an amount comprised between 1 mg and 1000 mg, preferably between 10 and 700 mg, still more preferably between 50 and 300 mg.

In the present context, the extract of a plant of genus *Cynara* is an aqueous extract of leaves of *Cynara cardunculus,* var. *scolymus* and *altilis.* The extract appears as a yellow-brown powder, with a sour taste and a characteristic odor. The extract preferably contains a concentration by weight of luteolin between 2 and 4% w/w and a concentration by weight of chlorogenic acid and derivatives between 10 and 12% w/w.

A non-limiting example of extract of plant belonging to the genus *Cynara* is the extract of *Cynara cardunculus* (var. *altilis* and *scolymus)* marketed by Bionap S.r.l.

In the present context, the extract of *Fraxinus excelsior* is obtained by hot water extraction of leaves, with a ratio E/D (Extract/Drug) of 1/3-4 and titrated in total hydroxycinnamic derivatives, expressed as chlorogenic acid ≥ 2% w/w (spectrophotometric method, Ph Eur). The extract is dried through a vacuum dryer and it appears as a hygroscopic powder and moderately soluble in water, brown in color, with a characteristic taste and odor.

For example, the dry extract (d.e.) of 2% *Fraxinus excelsior* (L.) marketed by Farmalabor Srl can be used.

Suitable acceptable carriers are those commonly known to the man skilled in the art for the preparation of pharmaceutical compositions for oral administration such as powders, granulates, capsules, tablets, solutions, suspensions, etc. By way of non-limiting example, said acceptable carriers can consist of diluents (for example dibasic calcium phosphate, lactose, microcrystalline cellulose and cellulose derivatives), thickeners (for example rubbers, hydroxypropyl methylcellulose and other cellulose derivatives), sweeteners (for example sorbitol, mannitol and other polyols, acesulfame K, aspartame, cyclamates, saccharin, sucralose), lubricants (for example magnesium stearate, stearic acid, waxes), dispersants, surfactants (for example sodium lauryl sulfate and polysorbates), flavoring agents, adsorbents (for example silica gel, talc, starch, bentonite, kaolin), glidants and non-sticking agents (for example talc, colloidal silica, corn starch, silicon dioxide), dyes (for example iron oxides), matting agents (for example titanium oxide), antioxidants, binders (for example rubbers, starch, jelly, cellulose derivatives, sucrose, sodium alginate), disintegrants (starch, microcrystalline cellulose, alginic acid, crospovidone), plasticizers (for example ethyl cellulose and other cellulose derivatives, acrylates and methacrylates, glycerol and sorbitol), preservatives (for example parabens, sulfur dioxide), thickeners, emulsifiers, humectants, wetting agents, chelating agents and mixture thereof.

The composition object of the present invention can be a medical device, a food supplement, a nutraceutical, dietetic and nutritional composition, a food product, a beverage, a nutraceutical product, a medicament, a medicated food, a pharmaceutical composition or a food for special medical purposes.

The composition object of the present invention is preferably a solid, semisolid or liquid pharmaceutical composition for oral use.

In an embodiment, the composition object of the present invention is in form of a powder, granulate, rigid capsule, soft-gel capsule, tablet or sachet. In another embodiment, the composition object of the present invention is in the form of a solution, suspension or emulsion.

The compositions of the present invention is particularly effective to reduce plasma levels of uric acid thanks to the synergistic action of their components.

The composition object of the present invention exhibits a valid synergistic effect, in particular, when the extract of plant belonging to the genus *Cynara* is in an amount comprised between 1 mg and 5000 mg and the extract of plant belonging to the genus *Fraxinus* is in an amount comprised between 1 mg and 1000 mg.

More in particular, the composition object of the present invention exhibits a valid synergistic effect when the extract of *Cynara cardunculus* (var. *scolymus* and/or *altilis*) is in an amount comprised between 1 mg and 5000 mg and the extract of *Fraxinus excelsior* is in an amount comprised between 1 mg and 1000 mg.

Therefore, a further object of the present invention is a composition comprising extract of *Cynara scolymus* and/or *altilis* and extract of *Fraxinus excelsior* in admixture with one or more suitable available carriers for the use in reducing plasma levels of uric acid and, therefore, in the treatment of hyperuricemia and renal or cardiovascular diseases related to hyperuricemia.

Non-limiting examples of renal diseases related to hyperuricemia are kidney stones and kidney failure, whereas non-limiting examples of cardiovascular diseases associated to hyperuricemia are hypertension, coronary heart diseases, peripheral vascular diseases and ischemia.

Moreover, a further object of the present invention is a composition comprising extract of *Cynara scolymus* and/or *altilis* and extract of *Fraxinus excelsior* in admixture with one or more suitable available carriers for use in the treatment of gout.

Without being bound to a specific theory, the inventors believe that the effect of the association extract of at least a plant belonging to the genus *Cynara* and extract of at least a plant belonging to the genus *Fraxinus,* according to the present invention, derives from the following activities of the components of the association.

### Reduction of hyperuricemia

The effectiveness of the composition object of the present invention is evaluated according to the experimental protocols known to the man skilled in the art. In particular, the effectiveness of the individual components and their association in inhibiting the xanthine oxidase enzyme has been evaluated *in vitro.* The ability to inhibit the activity of xanthine oxidase has been determined by calculating with spectrophotometry, at a wave length of 295 nm, the concentration of uric acid produced in the absence or in the presence of individual components and their association.

The reaction mixture consisted of 80 mM sodium pyrophosphate buffer (pH 7.5), 120 mM xhantine and 0.1 U of enzyme. The absorption trend, indicative of uric acid formation, was monitored at 295 nm, and the initial speed was calculated. The aqueous extracts of *Cynara scolymus* and *Fraxinus excelsiorwere* solubilized directly in the buffer. All the determinations were executed in triplicate. The inhibitory activity of each extract and of their association was calculated at increasing concentrations.

### EXAMPLES

By way of example are now provided some non-binding examples of daily doses of active components of the composition object of the present invention. Daily doses are meant to be administrated in a suitable oral dosage form and divided in one or more dosage units, such as, for example, a capsule, a tablet or a sachet.

Changes and variations of the embodiments herein exemplified, obvious to a person skilled in the art, are included in the appended claims.

### EXAMPLE 1

| *Active Ingredient* | *Daily dose* |
|---|---|
| Fraxinus excelsior, d.e. | 150 mg |
| Cynara cardunculus, d.e. | 100 mg |

### EXAMPLE 2

| *Active Ingredient* | *Daily dose* |
|---|---|
| Cynara cardunculus, d.e. | 500 mg |
| Fraxinus excelsior, d.e. | 150 mg |

### EXAMPLE 3

| *Active Ingredient* | *Daily dose* |
|---|---|
| Cynara cardunculus, d.e. | 300 mg |
| Fraxinus excelsior, d.e. | 100 mg |

### EXAMPLE 4

| *Active Ingredient* | *Daily dose* |
|---|---|
| Cynara cardunculus, d.e. | 500 mg |
| Fraxinus excelsior, d.e. | 100 mg |

### EXAMPLE 5

| *Active Ingredient* | *Daily dose* |
|---|---|
| Cynara cardunculus, d.e. | 2000 mg |
| Fraxinus excelsior, d.e. | 200 mg |

### EXAMPLE 6

| *Active Ingredient* | *Daily dose* |
|---|---|
| Cynara cardunculus, d.e. | 2000 mg |
| Fraxinus excelsior, d.e. | 100 mg |

### EXAMPLE 7

| *Active Ingredient* | *Daily dose* |
|---|---|
| Cynara cardunculus, d.e. | 1000 mg |
| Fraxinus excelsior, d.e. | 100 mg |

### EXAMPLE 8: Pharmaceutical form Capsule of 880 mg

| *Active Ingredient* | *Amount* |
|---|---|
| Cynara cardunculus, d.e. | 500 mg |
| Fraxinus excelsior, d.e. | 150 mg |
| *Excipients* | |
| Microcrystalline cellulose | 100 mg |
| Silicon dioxide | 25 mg |

| *Casing* | |
|---|---|
| Jelly | 67.5 mg |
| Glycerol | 15 mg |
| Water | q.s. |
| Titanium Dioxide (E171) | 5 mg |
| Curcumin (E100) | 2 mg |

The formulation of Example 8 was prepared by mixing the extract of *Cynara cardunculus* (var. *scolymus)* and the extract of *Fraxinus excelsior* with the excipients and by inserting said mixture in a capsule with a capsule filling machine.

### EXAMPLE 9: Pharmaceutical form Tablet of 997 mg

| *Active Ingredient* | *Amount* |
|---|---|
| Cynara cardunculus, d.e. | 500 mg |
| Fraxinus excelsior, d.e. | 100 mg |
| *Excipients* | |
| Microcrystalline cellulose | 300 mg |
| Silicon dioxide | 50 mg |
| Polysorbate 80 | 20 mg |
| Magnesium stearate | 5 mg |
| (*Coating agents*) | |
| Hydroxypropylcellulose | 15 mg |
| Titanium Dioxide (E171) | 5 mg |
| Curcumin (E100) | 2 mg |

The formulation of Example 9 was prepared by mixing the extract of *Cynara cardunculus* (var. *scolymus*) and the extract of *Fraxinus excelsior* with the excipients and by compressing said mixture, with a tableting machine, to form a tablet. The tablet was subsequently coated with a film of hydroxypropylcellulose, Titanium Dioxide and Curcumin (E100).

### EXAMPLE 10: Pharmaceutical form Sachet of 2315 mg

| *Active Ingredient* | *Amount* |
|---|---|
| Cynara cardunculus, d.e. | 1000 mg |
| Fraxinus excelsior, d.e. | 300 mg |
| *Excipients* | |
| Maltodextrin | 500 mg |
| Fructose | 300 mg |
| Silicon dioxide | 200 mg |
| Flavour | 15 mg |

The formulation of Example 10 was prepared by mixing the extract of *Cynara cardunculus* (var. *scolymus*) and the extract of *Fraxinus excelsior* with the excipients and by inserting this mixture in a sachet.

## Claims

1. A composition comprising an association of extract of at least a plant belonging to the genus *Cynara* in an amount between 1 mg and 5000 mg and extract of at least a plant belonging to the genus *Fraxinus* in an amount between 1 mg and 1000 mg in admixture with one or more suitable acceptable carriers wherein the extract of at least a plant belonging to the genus *Cynara* is an aqueous extract of leaves of *Cynara cardunculus var. scolymus and altilis* containing a concentration by weight of luteolin between 2 and 4% w/w and a concentration by weight of chlorogenic acid between 10 and 12% w/w, and wherein the extract of at least a plant belonging to the genus *Fraxinus* is of *Fraxinus excelsior,* and obtainable by hot water extraction of leaves with a ratio Extract/Drug of 1/3-4 and titrated in total hydroxycinnamic derivatives, expressed as chlorogenic acid ≥ 2% w/w, spectrophotometric method (Ph.Eur).

2. Composition according to any of the previous claims, wherein the composition is a medical device, a food supplement, a nutraceutical, dietetic and nutritional composition, a food product, a beverage, a nutraceutical product, a medicament, a medicated food, a pharmaceutical composition or a food for special medical purposes.

3. Composition according to any of the previous claims, in the form of a liquid, solid or semisolid composition for oral use.

4. Composition according to claim 3 in the form of powder, granulate, rigid capsule, softgel capsule, tablet, sachet, solution, suspension or emulsion.

5. Composition according to any of the previous claims for use in the prevention or in the treatment of hyperuricemia and of renal or cardiovascular diseases related to hyperuricemia.

6. Composition according to claim 5 for use in the treatment of gout.

## Patentansprüche

1. Zusammensetzung, umfassend eine Assoziation von Extrakt mindestens einer Pflanze, die zu der Gattung *Cynara* gehört, in einer Menge zwischen 1 mg und 5000 mg und Extrakt mindestens einer Pflanze, die zu der Gattung *Fraxinus* gehört, in einer Menge zwischen 1 mg und 1000 mg unter Beimischung von einem oder mehreren geeigneten unbedenklichen Trägerstoffen, wobei der Extrakt mindestens einer Pflanze, die zu der Gattung *Cynara* gehört, ein wässriger Extrakt aus Blättern von *Cynara cardunculus var. scolymus und altilis* ist, der eine Gewichtskonzentration von Luteolin zwischen 2 und 4 Gew.-% und eine Gewichtskonzentration von Chlorogensäure zwischen 10 und 12 Gew.-% enthält, und wobei der Extrakt mindestens einer Pflanze, die zu der Gattung *Fraxinus* gehört, von *Fraxinus excelsior* stammt und durch Heißwasserextraktion von Blättern mit einem Droge-Extrakt-Verhältnis von 3-4/1 und titriert in Gesamt-Hydroxyzimtderivaten, ausgedrückt als Chlorogensäure ≥ 2 Gew.-%, spektrophotometrisches Verfahren (Ph.Eur), erhaltbar ist.

2. Zusammensetzung nach einem der vorhergehenden Ansprüche, wobei die Zusammensetzung ein Medizinprodukt, ein Nahrungsergänzungsmittel, eine nutrazeutische, diätetische und Ernährungszusammensetzung, ein Lebensmittelprodukt, ein Getränk, ein nutrazeutisches Produkt, ein Medikament, ein medizinisches Lebensmittel, eine pharmazeutische Zusammensetzung oder ein Lebensmittel für spezielle medizinische Zwecke ist.

3. Zusammensetzung nach einem der vorhergehenden Ansprüche in der Form einer flüssigen, festen oder halbfesten Zusammensetzung zur oralen Anwendung.

4. Zusammensetzung nach Anspruch 3 in der Form von Pulver, Granulat, Hartkapsel, Weichgelkapsel, Tablette, Beutel, Lösung, Suspension oder Emulsion.

5. Zusammensetzung nach einem der vorhergehenden Ansprüche zur Verwendung bei der Vorbeugung oder bei der Behandlung von Hyperurikämie und von mit Hyperurikämie verbundenen Nieren- oder Herz-Kreislauf-Erkrankungen.

6. Zusammensetzung nach Anspruch 5 zur Verwendung bei der Behandlung von Gicht.

## Revendications

1. Composition comprenant une association d'un extrait d'au moins une plante appartenant au genre *Cynara* en une quantité comprise entre 1 mg et 5000 mg et d'un extrait d'au moins une plante appartenant au genre *Fraxinus* en une quantité comprise entre 1 mg et 1000 mg en mélange avec un ou plusieurs vecteurs acceptables appropriés, ledit extrait d'au moins une plante appartenant au genre *Cynara* étant un extrait aqueux de feuilles de *Cynara cardunculus var. scolymus et altilis* contenant une concentration en poids de lutéoline comprise entre 2 et 4 % en p/p et une concentration en poids d'acide chlorogénique comprise entre 10 et 12 % en p/p, et ledit extrait d'au moins une plante appartenant au genre *Fraxinus* étant de *Fraxinus excelsior,* et pouvant être obtenu par extraction à l'eau chaude des feuilles avec un rapport Extrait/Médicament de 1/3 à 4 et titré en dérivés hydroxycinnamiques totaux, exprimés en acide chlorogénique ≥ 2 % en p/p, méthode spectrophotométrique (Ph.Eur).

2. Composition selon l'une quelconque des revendications précédentes, ladite composition étant un dispositif médical, un complément alimentaire, une composition nutraceutique, diététique et nutritionnelle, un produit alimentaire, une boisson, un produit nutraceutique, un médicament, un aliment médicamenteux, une composition pharmaceutique ou un aliment destiné à des fins médicales spéciales.

3. Composition selon l'une quelconque des revendications précédentes sous la forme d'une composition liquide, solide ou semi-solide à usage oral.

4. Composition selon la revendication 3 sous la forme d'une poudre, d'un granulé, d'une capsule rigide, d'une capsule molle, d'un comprimé, d'un sachet, d'une solution, d'une suspension ou d'une émulsion.

5. Composition selon l'une quelconque des revendications précédentes destinée à être utilisée dans la prévention ou dans le traitement de l'hyperuricémie et des maladies rénales ou cardiovasculaires liées à l'hyperuricémie.

6. Composition selon la revendication 5 destinée à être utilisée dans le traitement de la goutte.
